# EUROPEAN PATENT SPECIFICATION

(11) **EP 2 906 181 B1**
(45) Date of publication and mention of the grant of the patent: **06.12.2017**
(21) Application number: 13766295.3
(22) Date of filing: 23.09.2013
(51) Int. Cl.: A61K 8/19, A61K 8/25, A61Q 11/00, A61K 8/02

(54) **ORAL CARE COMPOSITION**
MUNDPFLEGEMITTEL
COMPOSITION DE PROTECTION ORALE

(30) Priority: 12.10.2012 WO PCT/CN2012/082837; 16.11.2012 EP 12193013
(43) Date of publication of application: 19.08.2015
(73) Proprietor: Unilever N.V., 3013 AL Rotterdam (NL); Unilever PLC, London, Greater London EC4Y 0DY (GB)
(72) Inventor: DING, Guanjun, Shanghai 200335 (CN); LI, Xiaoke, Shanghai 200335 (CN); LIU, Weining, Shanghai 200335 (CN); XIANG, Xing, Shanghai 200051 (CN)
(74) Representative: Tansley, Sally Elizabeth
(86) International application number: PCT/EP2013/069714
(87) International publication number: WO 2014/056713

(56) References cited:
- US-A- 4 420 312
- US-A- 4 486 404
- US-A1- 2002 037 258
- US-A1- 2005 123 490
- US-A1- 2006 110 306
- US-A1- 2010 136 067
- US-A1- 2012 141 588

## Description

### TECHNICAL FIELD OF THE INVENTION

The present invention relates to oral care compositions such as tooth pastes, gums, mouthwashes and the like. In particular the present invention relates to oral care compositions containing calcium silicate hydrate. The invention also relates to the use of such compositions for remineralizing and/or whitening of teeth of an individual and to processes for manufacturing the compositions.

### BACKGROUND TO THE INVENTION

Products we enjoy as consumers, unfortunately, often have a negative impact on our teeth. Acidic drinks and sweets, for example, can result in tooth erosion by attacking enamel that coats and protects the teeth. Moreover, tobacco based products as well as beverages like coffee and tea can stain teeth and thereby result in a smile that is often not attractive.

In addition to what we consume, the natural equilibrium between tooth hydroxyapatite (HAP) being dissolved from the enamel of teeth and HAP being formed on or in teeth from substances occurring naturally in the saliva shifts continuously. Such a shift can yield unattractive teeth with cariogenic conditions. Products that address tooth decay and/or whitening have, nevertheless, been developed, including products that employ calcium salts for remineralization of teeth.

WO 2008/068149 A (Unilever) discloses an oral care product comprising a first composition comprising an insoluble calcium salt that is not a calcium phosphate salt, a second independent composition comprising a source of phosphate ions, and a means for delivering each of the compositions to the surface of the teeth. The preferred insoluble calcium salt is calcium silicate.

WO 2008/015117 A (Unilever) discloses a calcium oxide-silica composite biomaterial either in amorphous state or crystalline state having an average pore size, as determined by the BET method, in the range of from 0.8 to 4 nm, wherein the calcium oxide-silica content of the biomaterial is at least 80 wt percent, the balance being optionally one or more other materials and wherein the molar ratio of calcium oxide to silica is at least 0.1. US2002/0037258 discloses dental products for sensitive teeth using amorphous particulate material comprising calcium silicate. We have now found that providing calcium silicate in the form of a hydrate unexpectedly enhances its performance in terms of providing oral care benefits. If used in amounts of 10-40%. In particular, calcium silicate hydrate is found to effectively deposit on teeth surfaces from oral care compositions, even where such compositions are substantially free from phosphate. In addition, calcium silicate hydrate is found to provide oral care benefits without necessarily having a specific structure (such as being mesoporous) thus allowing for more convenient manufacture. The scope of the invention is defined by the appended claims.

### TESTS AND DEFINITIONS

### Dentifrice

"Dentifrice" for the purposes of the present invention means a paste, powder, liquid, gum or other preparation for cleaning the teeth or other surfaces in the oral cavity.

### Tooth Paste

"Tooth paste" for the purposes of the present invention means a paste or gel dentifrice for use with a toothbrush. Especially preferred are tooth pastes suitable for cleaning teeth by brushing for about two minutes.

### Mouth Wash

"Mouth wash" for the purposes of the present invention means liquid dentifrice for use in rinsing the mouth. Especially preferred are mouth washes suitable for rinsing the mouth by swishing and/or gargling for about half a minute before expectorating.

### Particle Size

Particle size as used herein refers to particle diameter unless otherwise stated. Diameter is meant to mean the largest measurable distance on a particle in the event a well-defined sphere is not generated. For polydisperse samples, diameter means the z-average particle size measured, for example, using dynamic light scattering (see international standard ISO 13321) with an instrument such as a Zetasizer Nano™ (Malvem Instruments Ltd, UK).

### pH

pH is quoted at atmospheric pressure and a temperature of 25 °C. When referring to the pH of an oral care composition, this means the pH measured when 1 part by weight of the composition is uniformly dispersed and/or dissolved in 20 parts by weight pure water at 25 °C. In particular the pH may be measured by manually mixing 1 g oral care composition with 20 ml water for 30 s, then immediately testing the pH with indicator paper or a pH meter.

### Solubility

"Soluble" and "insoluble", as used herein, refers to the solubility of a source (e.g., like calcium salts) in water at 25 °C and atmospheric pressure. "Soluble" means a source that dissolves in water to give a solution with a concentration of at least 0.1 moles per litre. "Insoluble" means a source that dissolves in water to give a solution with a concentration of less than 0.001 moles per litre. "Sparingly soluble", therefore, is defined to mean a source that dissolves in water to give a solution with a concentration of greater than 0.001 moles per litre and less than 0.1 moles per litre.

### Water of Hydration

"Water of hydration", as used herein, refers to water chemically bound to a substance in a way that it can only be removed by heating without substantially changing the chemical composition of the substance. In particular, water which could only be removed when heated above 200°C. The water loss is measured using thermo gravimetric analysis (TGA) with a Netzsch TG instrument. The TGA is conducted under an N₂ atmosphere with heating rate of 10 degree/min in the range of 30 to 900°C.

### Substantially Free

"Substantially free of', as used herein, means less than 1.5%, and preferably less than 1.0%, and more preferably less than 0.75% and more preferably still less than 0.5%, and even more preferably less than 0.1% and most preferably from 0.0 to 0.01 % by weight, based on total weight of the oral care composition, including all ranges subsumed therein.

### Viscosity

Viscosity of a tooth paste is the value taken at room temperature (25 °C) with a Brookfield Viscometer, Spindle No. 4 and at a speed of 5 rpm. Values are quoted in centipoises (cP = mPa.s) unless otherwise specified.

### Remineralization

"Remineralization", as used herein, means *in situ* (i.e. in the oral cavity) generation of calcium phosphate on teeth (including layers on teeth from 10 nm to 20 microns, and preferably from 75 nm to 10 microns, and most preferably, from 150 nm to 5 microns thick including all ranges subsumed therein) to reduce the likelihood of tooth sensitivity, tooth decay, regenerate enamel and/or improve the appearance of teeth by whitening through the generation of such new calcium phosphate.

### Miscellaneous

Except in the examples, or where otherwise explicitly indicated, all numbers in this description indicating amounts of material or conditions of reaction, physical properties of materials and/or use may optionally be understood as modified by the word "about".

All amounts are by weight of the final oral care composition, unless otherwise specified.

It should be noted that in specifying any range of values, any particular upper value can be associated with any particular lower value.

For the avoidance of doubt, the word "comprising" is intended to mean "including" but not necessarily "consisting of' or "composed of'. In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

### SUMMARY OF THE INVENTION

In a first aspect, the present invention is directed to an oral care composition comprising:
a) from 10-40% by weight calcium silicate hydrate comprising from 20 to 70% SiO₂ by weight of the calcium silicate hydrate; and
b) physiologically acceptable carrier comprising humectant, surfactant,
thickener or a mixture thereof, wherein the calcium silicate hydrate comprises water of hydration in an amount of from 5 to 40% by weight of the calcium silicate hydrate. In a second aspect, the invention is directed to the use of the composition in a therapeutic method of remineralizing and/or whitening of teeth of an individual comprising the step of applying the oral care composition of any embodiment of the first aspect to one or more teeth of the individual.

In a third aspect, the present invention is directed to process for manufacturing an oral care composition as claimed in any of claims 1 to 8 comprising the steps of:
i) contacting a source of silicate ions with a source of calcium ions in an aqueous medium to form a reaction mixture;
ii) recovering calcium silicate hydrate from the reaction mixture;
iii) combining the calcium silicate hydrate with physiologically acceptable carrier.

Thus the present invention also provides the oral care composition of the first aspect obtained and/or obtainable by the process of the third aspect of the invention. All other aspects of the present invention will more readily become apparent upon considering the detailed description and examples which follow.

### BRIEF DESCRIPTION OF THE FIGURES

Fig.1 shows the XRD pattern of a calcium silicate hydrate powder suitable for use in an embodiment of the invention.
Fig. 2 shows the small angle XRD pattern of the material of Fig. 1.
Fig. 3 shows the results of TGA of the material of Fig. 1.

### DETAILED DESCRIPTION

The calcium silicate hydrate for use in the present invention comprises at least calcium oxide (CaO), silica (SiO₂) and water.
The present inventors have found that the ability of the calcium silicate hydrate to remineralize dental tissue is improved with increasing amounts of silica content. Thus the calcium silicate hydrate comprises at least 20% silica by weight of the calcium silicate hydrate, preferably at least 30%, more preferably at least 40% and most preferably at least 55%.

The present inventors have also found that, compared with some conventional calcium silicates which are not hydrated, calcium silicate hydrate shows enhanced deposition on dental tissue. Thus, to allow for the presence of water and calcium oxide (and additional components, if desired) the silica content of the calcium silicate hydrate is no greater than 70% by weight of the calcium silicate hydrate, preferably no greater than 65% and most preferably no greater than 60%.

The calcium silicate hydrate preferably comprises the water of hydration in an amount of at least 5% by weight of the calcium silicate hydrate, more preferably at least 10%, more preferably still at least 15%, even more preferably at least 20% and most preferably at least 25%. The water content is typically no greater than 50% by weight of the calcium silicate hydrate, more preferably no greater than 40%, even more preferably no greater than 35% and most preferably no greater than 30%.

To provide calcium necessary for remineralization, the calcium silicate hydrate preferably comprises calcium oxide in an amount of at least 5% by weight of the calcium silicate hydrate, more preferably at least 7%, more preferably still at least 10%, even more preferably at least 12% and most preferably at least 15%. The CaO content is typically no greater than 50% by weight of the calcium silicate hydrate, more preferably no greater than 40%, even more preferably no greater than 30% and most preferably no greater than 25%.

A convenient measure of the relative proportions of calcium oxide and silica in the calcium silicate hydrate is the atom ratio of Calcium (Ca) to Silicon (Si). As stated above, the present inventors have found that the ability of the calcium silicate hydrate to remineralize dental tissue is improved with increasing amounts of silica content. Thus the calcium silicate hydrate preferably comprises Ca and Si in an atom ratio (Ca:Si) less than 1:1, more preferably less than 1:1.2, more preferably still from 1:1.5 to 1:4 and most preferably from 1:1.7 to 1:3.

The calcium silicate hydrate may be amorphous. However the present inventors have surprisingly found that even if the calcium silicate is at least partly crystalline, it may still function effectively to remineralize dental tissue. Thus preferably the calcium silicate hydrate is at least partly crystalline. The presence of crystallinity may be confirmed, for example, by x-ray diffraction.

The calcium silicate hydrate may be mesoporous. Mesoporous calcium silicate hydrate is described, for example, in J. Wu et al. "Hierachically Nanostructured Mesoporous Spheres of Calcium Silicate Hydrate: Surfactant-Free Sonochemical Synthesis and Drug-Delivery System with Ultrahigh Drug-Loading Capacity", Advanced Materials, 2010, 22, pp. 749-753. However, the present inventors have found that even in the absence of mesopores, calcium silicate hydrate can effectively deposit on and remineralize dental tissue. Without wishing to be bound by theory, the inventors believe that this could be owing to the presence of water of hydration which makes the calcium silicate more reactive. Therefore preferably the calcium silicate hydrate is not mesoporous. The presence or absence of mesopores can be confirmed, for example, using small angle x-ray diffraction and/or nitrogen absorption-desorption.

The calcium silicate hydrate is preferably particulate as this allows for maximum surface area for contact with dental tissue. Thus preferably the composition comprises particles comprising the calcium silicate hydrate. More preferably the particles have a weight average particle size of five (5) microns or less, and even more preferably from 10 to 100%, and especially, from 25 to 100%, and most especially, from 70 to 100% by weight of the particles comprising calcium silicate hydrate used in this invention have a particle size from 0.1 to 1.5 microns.

In addition to calcium oxide, silica and water, the particles which comprise the calcium silicate hydrate may comprise other components, such as metal cations, anions (such as phosphate) and the like. However, it is preferred that the particles comprise CaO, SiO₂ and water in an amount of at least 70% by weight of the particles, more preferably at least 80%, more preferably still at least 90% and even more preferably at least 95%. Most preferably the particles consist of (or at least consist essentially of) CaO, SiO₂ and water.

Typically, the oral care composition of the present invention comprises from 0.1 to 60% by weight of the calcium silicate hydrate, more preferably from 0.2 to 50%. Even more preferably the composition comprises the calcium silicate hydrate in an amount of at least 0.3% by weight, more preferably still at least 0.5% or even at least 1%. In a most preferred embodiment the composition comprises the calcium silicate hydrate in an amount of at least 5% by weight, and optimally in the range 10 to 40% by weight of the oral care composition.

Where the composition is a tooth paste or powder, higher amounts of the calcium silicate hydrate are preferred. This is because tooth pastes are typically applied only in small volumes (e.g. around 2 ml) per consumer use. In addition tooth pastes are typically opaque and therefore allow for incorporation of high levels of calcium silicate hydrate without affecting the appearance expected by the consumer. Thus in one embodiment, the composition is a tooth paste or powder and comprises the calcium silicate hydrate in an amount of at least 2% by weight, more preferably at least 5% by weight and most preferably in the range 7 to 40% by weight.

Where the composition is a mouth wash, lower amounts of the calcium silicate hydrate are preferred. This is because mouth washes are typically used in larger volumes (e.g. around 20 ml) per consumer use than tooth pastes. In addition mouth washes are typically transparent and therefore incorporation of high levels of calcium silicate hydrate may negatively affect the appearance expected by the consumer. Thus in one embodiment, the composition is a mouth wash and comprises the calcium silicate hydrate in an amount of at least 0.2% by weight, more preferably at least 0.5% by weight and most preferably from 1 to 10% by weight.

The composition of the present invention is found to be capable of remineralisation of teeth *in situ* without inclusion of a phosphate source in the composition itself. Without wishing to be bound by theory the present inventors believe that this may be because the calcium source in the composition of the invention reacts with phosphate ions in saliva and/or Si-OH groups may have an affinity for Ca ions in teeth.

Thus in one embodiment the composition may be substantially free of phosphate source. This is especially preferred when the composition is a monophase hydrous composition (i.e. comprises greater than 1.5% water, preferably greater than 5% water, more preferably greater than 10% water and most preferably from 20 to 90% water by weight of the composition). Presence of both the calcium silicate hydrate and phosphate sources in a monophase hydrous formulation can lead to premature reaction of the calcium and phosphate and instability of the product.

For certain compositions, especially anhydrous compositions (i.e. compositions substantially free from water) or dual phase hydrous compositions, it is preferable to compound a phosphate source in the composition to aid *in situ* generation of calcium phosphate.

The phosphate source that may be used in this invention is limited only to the extent that the same may be used in a composition suitable for use in an oral cavity. Illustrative examples of the types of phosphate source suitable for use in this invention include trisodium phosphate, monosodium dihydrogen phosphate, disodium hydrogen phosphate, sodium pyrophosphate, tetrasodium pyrophosphate, sodium hexametaphosphate, tripotassium phosphate, monopotassium dihydrogen phosphate, dipotassium hydrogen phosphate, mixtures thereof or the like. The phosphate source is preferably one which is water soluble.

Typically, the phosphate source makes up from 0.5 to 15%, and more preferably from 2 to 12%, and most preferably from 4 to 9% by weight of the oral care composition, based on total weight of the composition and including all ranges subsumed therein. In a most preferred embodiment, the phosphate source used is trisodium phosphate and monosodium dihydrogen phosphate at a trisodium phosphate to monosodium dihydrogen phosphate weight ratio of 1:4 to 4:1, preferably 1:3 to 3:1, and most preferably, from 1:2 to 2:1, including all ratios subsumed therein.

The oral care composition preferably has a pH of greater than 5.0. If the pH of the composition is too low then it may lower the pH in the oral cavity such that generation of *in situ* calcium phosphate is retarded. Therefore it is preferred that the pH of the oral care composition is in the range 5.5 to 11.0, more preferably 6.0 to 10.5 and most preferably 7.0 to 10.0.

The composition of the present invention is an oral care composition and so comprises physiologically acceptable carrier. The carrier comprises at least surfactant, thickener, humectant or a combination thereof.

Preferably the oral care composition comprises a surfactant. Preferably the composition comprises at least 0.01 % surfactant by weight of the composition, more preferably at least 0.1% and most preferably from 0.5 to 7%. Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used. More preferably the surfactant comprises or is anionic surfactant. The preferred anionic surfactants are sodium lauryl sulphate and/or sodium dodecylbenzene sulfonate. Most preferably the surfactant is sodium lauryl sulphate.

Thickener may also be used in this invention and is limited only to the extent that the same may be added to a composition suitable for use in the mouth. Illustrative examples of the types of thickeners that may be used in this invention include, sodium carboxymethyl cellulose (SCMC), hydroxyl ethyl cellulose, methyl cellulose, ethyl cellulose, gum tragacanth, gum arabic, gum karaya, sodium alginate, carrageenan, guar, xanthan gum, Irish moss, starch, modified starch, silica based thickeners including silica aerogels, magnesium aluminum silicate (e.g., Veegum), Carbomers (cross-linked acrylates) and mixtures thereof.

Typically, sodium carboxymethyl cellulose and/or a Carbomer is/are preferred, most preferably sodium carboxymethyl cellulose.

When a Carbomer is employed, those having a weight-average molecular weight of at least 700,000 are desired, and preferably, those having a molecular weight of at least 1,200,000, and most preferably, those having a molecular weight of at least about 2,500,000 are desired. Mixtures of Carbomers may also be used herein.

In an especially preferred embodiment, the Carbomer is Synthalen PNC, Synthalen KP or a mixture thereof. It has been described as a high molecular weight and cross-linked polyacrylic acid and identified via CAS number 9063-87-0. These types of materials are available commercially from suppliers like Sigma.

In another especially preferred embodiment, the sodium carboxymethyl cellulose (SCMC) used is SCMC 9H. It has been described as a sodium salt of a cellulose derivative with carboxymethyl groups bound to hydroxy groups of glucopyranose backbone monomers and identified via CAS number 9004-32-4. The same is available from suppliers like Alfa Chem.

Thickener typically makes up from 0.01 to about 10%, more preferably from 0.1 to 9%, and most preferably, from 1.5 to 8% by weight of the oral care composition, based on total weight of the composition and including all ranges subsumed therein.

When the oral care composition of this invention is a toothpaste or gel, the same typically has a viscosity from about 30,000 to 180,000 centipoise, and preferably, from 60,000 to 170,000 centipoise, and most preferably, from 65,000 to 165,000 centipoise.

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants.

The humectant may be present in the range of from 10 to 90% by weight of the oral care composition. More preferably, the carrier humectant makes up from 25 to 80%, and most preferably, from 45 to 70% by weight of the composition, based on total weight of the composition and including all ranges subsumed therein.

Oral care compositions described herein may comprise optional ingredients which are common in the art. These ingredients include antimicrobial agents, anti-inflammatory agents, anti-caries agents, plaque buffers, fluoride sources, vitamins, plant extracts, desensitizing agents, anti-calculus agents, biomolecules, flavors, proteinaceous materials, preservatives, opacifying agents (especially titanium dioxide), coloring agents, pH-adjusting agents, sweetening agents, particulate abrasive materials, polymeric compounds, buffers and salts to buffer the pH and ionic strength of the compositions, and mixtures thereof. Such ingredients typically and collectively make-up less than 20% by weight of the composition, and preferably, from 0.0 to 15% by weight, and most preferably, from 0.01 to 12% by weight of the composition, including all ranges subsumed therein.

The oral care composition of this invention can be used in a method of remineralizing and/or whitening of teeth of an individual comprising the step of contacting one or more teeth of the individual with the oral care composition.

Typically the composition will be packaged. In tooth paste or gel form, the composition may be packaged in a conventional plastic laminate, metal tube or a single compartment dispenser. The same may be applied to dental surfaces by any physical means, such as a toothbrush, fingertip or by an applicator directly to the sensitive area. In liquid mouthwash form the composition may be packaged in a bottle, sachet or other convenient container.

The composition can be effective even when used in an individual's daily oral hygiene routine. For example, the composition may be brushed onto the teeth and/or be rinsed around the inside of the mouth of the individual. The composition may, for example, be contacted with the teeth for a time period of one second to 20 hours. More preferably from 10 s to 10 hours, more preferably still from 30 s to 1 hour and most preferably from 1 minute to 5 minutes. The composition may be used daily, for example for use by an individual once, twice or three times per day.

In a preferred embodiment the oral care composition is applied to the teeth without applying phosphate to the teeth (other than phosphate naturally present in the oral cavity).

Oral care compositions comprising calcium silicate hydrate may be manufactured by any convenient process but in a preferred embodiment, the oral care composition is manufactured by a process comprising the steps of:
i) contacting a source of silicate ions with a source of calcium ions in an aqueous medium to form a reaction mixture;
ii) recovering calcium silicate hydrate from the reaction mixture;
iii) combining the calcium silicate hydrate with physiologically acceptable carrier.

The amount of calcium source and silicate source in the reaction mixture can be varied to control the amount of CaO and SiO₂ in the final calcium silicate hydrate. The reaction mixture preferably comprises Ca and Si in an atom ratio (Ca:Si) less than 1:1, more preferably less than 1:1.2, more preferably still from 1:1.5 to 1:4 and most preferably from 1:1.7 to 1:3.

The source of silicate is preferably a water-soluble silicate salt, such as for example ammonium silicate, alkali metal silicate or a combination thereof. More preferably the silicate source is an alkali metal silicate, such as, for example, sodium silicate, potassium silicate or a combination thereof.

The source of calcium may, for example, be a soluble calcium salt such as calcium halide, calcium nitrate or the like. However, the present inventors have unexpectedly found that use of an insoluble or sparingly soluble calcium source produces calcium silicate hydrate which is easier to disperse than calcium silicate hydrate derived from soluble calcium source. Thus it is preferred that the source of calcium ions is selected from insoluble calcium source, sparingly soluble calcium source or a mixture thereof. Examples of sparingly soluble or insoluble calcium salts include, for example calcium hydroxide, calcium oxide, calcium acetate, calcium lactate, calcium gluconate, calcium carbonate, calcium sulphate, calcium carboxymethyl cellulose, calcium alginate, calcium salts of citric acid or a mixture thereof. Most preferably the calcium source is a sparingly soluble calcium salt, especially calcium hydroxide, calcium oxide or a mixture thereof.

The pH of the reaction mixture is preferably above pH 6.0 to ensure precipitation of solid calcium silicate hydrate from the reaction mixture and so allow for ease of separation. The present inventors have also found that the pH of the reaction mixture can influence the ability of the calcium silicate hydrate to remineralize dental tissue. Thus it is preferred that the pH of the reaction mixture is in the range of 8.0 to 12.5, more preferably 8.5 to 12.0.

The temperature of the reaction mixture may be any suitable temperature at which the calcium source and silicate source can react to form calcium silicate hydrate. However the present inventors have found that calcium silicate hydrate which is more easily dispersed in oral care formulations can be provided if the temperature is controlled in a specific range. Preferably the temperature of the reaction mixture is at least 10°C, more preferably at least 15°C and most preferably at least 20°C. It has been found that the reaction temperature does not need to be high to obtain calcium silicate hydrate. Typically, the reaction temperature of the reaction mixture is no greater than 70°C, more preferably no greater than 40°C, even more preferably no greater than 35°C and most preferably no greater than 30°C.

Preferably the reaction time is at least 1 hour, more preferably at least 2 hours and most preferably from 3 to 10 hours.

The step (ii) typically comprises a solid-liquid separation step. For example, the calcium silicate hydrate may be separated from the reaction mixture by a process selected from centrifugation, sedimentation, filtration, drying or a combination thereof. Solid calcium silicate hydrate recovered from the reaction mixture may then be washed with a solvent, especially a polar organic solvent (such as C₁-C₄ alcohol) or an aqueous solvent, more preferably an aqueous solvent, most preferably water.

Where the recovered calcium silicate hydrate is subjected to a drying step, it is preferred that the drying temperature is not too high as this could result in loss of water of hydration from the material. Thus it is preferred that the recovered calcium silicate hydrate is not heated to a temperature greater than 400 °C, more preferably not heated to a temperature greater than 300 °C, more preferably still not heated to a temperature greater than 200 °C and most preferably not heated to a temperature greater than 150 °C.

The step (iii) typically involves conventional formulation and mixing of ingredients to prepare an oral care formulation wherein such processes are well known to those skilled in the art.

The following examples are provided to facilitate an understanding of the present invention. The examples are not provided to limit the scope of the claims.

### EXAMPLES

### Example 1

This example demonstrates the improved deposition on dental tissue of calcium silicate hydrate compared to a commercial calcium silicate.

### Preparation of calcium silicate hydrate powder

The calcium silicate hydrate (CSH) was prepared using a mixture of calcium hydroxide and sodium silicate in deionised water. The mixture was formed with an initial Ca:Si ratio of 1:2. The mixture was continuously stirred whilst maintained at a temperature of 45 °C. The pH of the reaction mixture was adjusted to and maintained around 11 using hydrochloric acid. After stirring for 5 hours the reaction mixture was filtered and the cake washed three times with water before again being filtered. The filter cake was dried in an oven at 80 °C for 12 hours to yield the final CSH powder.

### Characterization of calcium silicate hydrate powder

The particle size of the CSH powder was measured using laser light diffraction (Malvern Mastersizer 2000 & Malvern ZetaSizer Nano series) and found to have an average size of 355 nm.

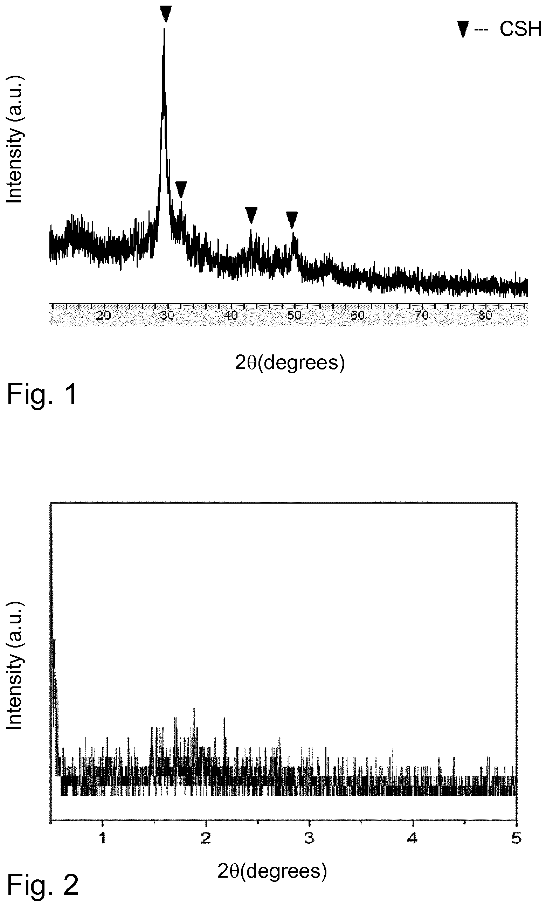

The powder x-ray diffraction (XRD) pattern of the material is shown in Fig. 1. Peaks are apparent in the pattern which indicates that the material was semi-crystalline.

The small angle XRD pattern of the material is shown in Fig. 2. No peaks are apparent in this pattern which indicates the absence of mesopores.

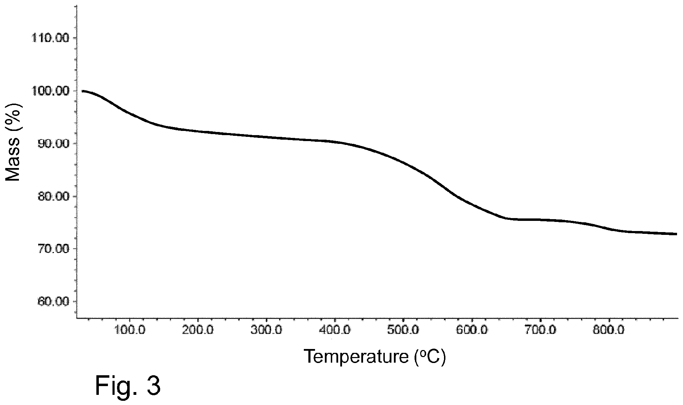

Mass loss was measured using thermo gravimetric analysis (TGA) with a Netzsch TG instrument. The TGA was conducted under an N₂ atmosphere with heating rate of 10 degree/min in the range of 30 to 900 °C. The results of are shown in Fig. 3. These results indicate that the material contained about 19 wt% water of hydration (i.e. water which could only be removed above 200 °C) and about 8 wt% of physically bound water (i.e. water which is removed under 200 °C).

### Deposition and Remineralization Evaluation

To evaluate deposition on tooth surface, the CSH powder was mixed with water at a ratio of 1.0 g powder to 10 ml water to make a slurry. Commercial calcium silicate (CaSiO₃, CS) supplied by P.Q. company (Microcal ET) was used as control.

Bovine enamel blocks were brushed with the fresh CSH-water slurry or CS-water slurry using a tooth brushing machine which was equipped with soft toothbrushes (Lion, Japan). The load of the tooth brushing was 170 g +/- 5 g and the automatic brushing was initiated at a speed of 150 rpm. After brushing for 1 min and incubation in the slurry for another 1 min, the tooth blocks were washed twice by distilled water (2 × 20 ml). Then, the tooth blocks were soaked in simulated oral fluid (SOF) at 37 °C under condition of shaking in a water bath. The tooth blocks were treated for 14 times to mimic one week of twice daily tooth brushing.

After one week treatment SEM images of the enamel block surfaces were taken. From the SEM images it was apparent that the CSH gave better and denser deposition on tooth surface than the commercial CS. From corresponding cross-section SEM images, it appeared that the commercial CS treated samples did not have a layer formed on their surface, while the CSH treated samples showed evidence of new layer formation on the surface. Analysis using EDX (Energy Dispersive X-ray Spectroscopy) indentified the elements of Si, Ca and P within the new layer, indicating that CSH deposited on tooth surface and induced biomineralization. Therefore, the CSH had enhanced deposition and remineralization efficacy as compared to the commercial CS.

### Example 2

This example demonstrates the effect of the relative amounts of CaO and SiO₂ on the properties of calcium silicate hydrate.

The calcium silicate hydrate was prepared using the same method as in Example 1 with different initial *Ca:Si* ratios of 2:1, 1:1 and 1:2.

The calcium silicate hydrates prepared with different initial *Ca:Si* ratios were each mixed with water at a ratio of 1.0 g / 10 ml. After one treatment with the CSH-water slurry under the same brushing procedure as in Example 1, the CSH with different initial *Ca:Si* ratios gave rise to similarly good and dense deposition on tooth surface as revealed by SEM analysis of brushed enamel blocks.

The obtained calcium silicate hydrate powder with different initial *Ca:Si ratios* was soaked in SOF at 37 °C under condition of shaking in a water bath. After soaking for 24 hours, the powder was dried and characterized by XRD to examine any composition changes. The XRD patterns of the samples with initial *Ca:Si* ratios of 2:1 and 1:1 showed peaks characteristic of calcium carbonate. As for the pattern of samples with an initial *Ca:Si* ratio of 1:2, these showed peaks characteristic of hydroxyapatite, indicating that the calcium silicate hydrate under such condition was bioactive in SOF and would be very likely to be able to induce the formation of hydroxyapatite *in situ.*

### Example 3

This example demonstrates the effect of the type of calcium source on the properties of calcium silicate hydrate.

### Preparation of calcium silicate hydrate powder starting from Calcium Nitrate

The calcium silicate hydrate (CSH) was prepared using a mixture of calcium nitrate and sodium silicate in deionised water. The mixture was formed with an initial Ca:Si ratio of 1:2. The mixture was continuously stirred whilst being maintained at a temperature of 45 °C. The pH of the reaction mixture was adjusted to and maintained around 11 using hydrochloric acid. After stirring for 5 hours the reaction mixture was filtered, washed once with ethanol, twice with water and then finally filtered. The filter cake was dried in an oven at 80 °C for 12 hours to yield the final CSH powder.

### Preparation of calcium silicate hydrate powder starting from Calcium Hydroxide

The CSH powder was prepared as described in Example 1.

### Comparison of properties

XRD results showed that calcium nitrate-derived reaction products were not purely CSH; while calcium hydroxide-derived reaction products were indeed substantially pure CSH. Furthermore, the calcium nitrate-derived powder size of 417 nm after water and ethanol washing treatment was still larger than the calcium hydroxide-derived powder size of 355 nm with only water washing treatment. Preparing CSH from calcium nitrate and substituting the ethanol wash for a water wash (as was done for the calcium hydroxide-derived powder) results in even larger particles.

### Example 4

This example demonstrates the effect of reaction pH on the properties of calcium silicate hydrate.

The calcium silicate hydrate was prepared using the same method as in Example 1, except that the pH was maintained at a value of 9.5, 11.7 or 13.1.

The CSH obtained under different pH was mixed with water at a ratio of 1.0 g / 10 ml. After one treatment with the CSH-water slurry under the same brushing procedure as in Example 1, the CSH obtained under different pH gave rise to similarly good and dense deposition on tooth surface as evidenced by SEM.

The calcium silicate hydrate powder obtained under different pH was soaked in SOF at 37 °C under condition of shaking in a water bath. After soaking for 24 hours, the powder was dried and characterized by XRD to test for compositional changes. Samples prepared at pH of 9.5 and 11.7 showed a tendency to convert into hydroxyapatite, indicating that the calcium silicate hydrate under such condition was bioactive in SOF and would be very likely to be able to induce the formation of hydroxyapatite *in situ.* As for samples prepared at pH of around 13.1, the calcium silicate hydrate did not transformed into hydroxyapatite in SOF, indicating that relatively high pH derived CSH had lower bioactivity in SOF.

### Example 5

This example demonstrates the effect of reaction time and temperature on the properties of calcium silicate hydrate.

The calcium silicate hydrate was prepared using the similar method as in Example 1 except that the reaction temperature was room temperature (25 °C) rather than 45 °C and the reaction time was 2 hours and 5 hours. It was found that the obtained CSH powder under different temperature had similarly good deposition on tooth surface. However, the powder obtained under conditions of room temperature and 2-hour stirring was not pure CSH, with other phases such as calcium carbonate. The powder obtained under conditions of room temperature (25°C) or 45°C with 5-hour stirring was pure and gave rise to a sole and characteristic pattern of CSH by XRD analysis. Therefore, increase of reaction time increased the purity of CSH powder.

### Example 6

This example demonstrates the effect of phosphate on deposition of calcium silicate hydrate.

The calcium silicate hydrate was prepared using the same method as in Example 1. To evaluate the effect of presence of phosphates on deposition of CSH on tooth surface, CSH-phosphate slurry and CSH-water slurry were made, respectively. The CSH-phosphate slurry was made by quickly mixing 1M K₂HPO₄, water and CSH with ratio of 1.5:10:1 (ml:ml:g). The CSH-water slurry was made by mixing CSH and water with a ratio of 1.0g/10ml. The tooth blocks were treated with the fresh slurry under the same procedures as in Example 1. The tooth blocks were treated for 14 times to mimic the one week of twice daily tooth brushing. The commercial CS was used as control. CS-phosphate slurry was made by mixing 1M K₂HPO₄, water and CS with ratio of 1.5:10:1 (ml:ml:g).

After one week treatment the CSH either with phosphates or not gave better and denser deposition on tooth surface than commercial CS as evidenced by SEM images of the surface of the tooth blocks. EDX analysis identified the elements of Si, Ca and P within the newly formed layers. Therefore, the CSH had enhanced deposition and remineralization efficacy as compared to the commercial CS and the presence of phosphates had little effect on deposition efficacy of CSH on tooth surface.

### Example 7

This example demonstrates toothpaste formulations according to the invention.

Anhydrous and hydrous mono-phase toothpaste formulations can be prepared as shown in Table 1.

**TABLE 1**

| **Component (%w/w)** | **Anhydrous Toothpaste** | **Hydrous Toothpaste** |
|---|---|---|
| Glycerine | To 100 | --- |
| PEG 400 | 10.50 | --- |
| Flavour | 1.20 | 1.00 |
| Trisodium Phosphate | 3.80 | --- |
| Calcium Silicate Hydrate* | 15.00 | 30.00 |
| Sodium Monofluorophosphate | 1.11 | 1.11 |
| Sweetener | 0.20 | 0.20 |
| Monosodium Dihydrogen Phosphate | 3.20 | --- |
| PEG 3000 | 1.75 | --- |
| Pigment | 0.05 | --- |
| Abrasive Silica | 7.00 | --- |
| Sodium Lauryl Sulphate | 2.00 | 2.20 |
| Sorbitol (70%) | --- | 20.0 |
| Water | --- | To 100 |
| Benz. Alcohol | --- | 0.50 |
| Potassium Nitrate | --- | 0.50 |
| Thickening Silica | --- | 1.00 |
| SCMC | --- | 0.30 |

| | | |
|---|---|---|
| *CSH powder from Example 1. | | |

## Claims

1. An oral care composition comprising:
a) from 10 to 40% by weight of calcium silicate hydrate comprising from 20 to 70% SiO₂ by weight of the calcium silicate hydrate; and
b) physiologically acceptable carrier comprising humectant, surfactant, thickener or a mixture thereof;
wherein the calcium silicate hydrate comprises water of hydration in an amount of from 5 to 40% by weight of the calcium silicate hydrate.

2. The oral care composition as claimed in claim 1, wherein the calcium silicate hydrate is particulate.

3. The oral care composition as claimed in claim 1 or claim 2, wherein the calcium silicate hydrate comprises from 5 to 40% CaO by weight of the calcium silicate hydrate.

4. The oral care composition as claimed in any one of the preceding claims, wherein the calcium silicate hydrate is not mesoporous.

5. The oral care composition as claimed in any one of the preceding claims, wherein the calcium silicate hydrate is at least partly crystalline.

6. The oral care composition as claimed in any one of the preceding claims, wherein the composition is a dentifrice, preferably a toothpaste or mouthwash.

7. The oral care composition as claimed in any one of the preceding claims, wherein the calcium silicate hydrate comprises Ca and Si in an atom ratio of from 1:1.5 to 1:3.

8. The oral care composition as claimed in any one of the preceding claims, wherein the oral care composition is a monophase composition and is substantially free from phosphate.

9. An oral care composition according to any one of claims 1 to 8 for use in a therapeutic method for remineralizing and/or whitening of teeth of an individual comprising the step of applying the oral care composition to one or more teeth of the individual.

10. The oral care composition for use in remineralizing and/or whitening of teeth of an individual, according to claim 9 wherein the oral care composition is applied to the teeth without applying phosphate to the teeth.

11. A process for manufacturing an oral care composition as claimed in any one of claims 1 to 8 comprising the steps of:
i) contacting a source of silicate ions with a source of calcium ions in an aqueous medium to form a reaction mixture;
ii) recovering calcium silicate hydrate from the reaction mixture;
iii) combining the calcium silicate hydrate with physiologically acceptable carrier,

12. The process as claimed in claim 11, wherein the reaction mixture is formed with a Ca:Si atom ratio of from 1:1.5 to 1:3.

13. The process as claimed in claim 11 or claim 12, wherein the source of silicate ions is a water soluble silicate salt, preferably an alkali metal silicate.

14. The process as claimed in any one of claims 11 to 13, wherein the source of calcium ions is selected from insoluble calcium salt, sparingly soluble calcium salt or a mixture thereof.

15. The process as claimed in claim 14, wherein the source of calcium ions is calcium hydroxide and/or calcium oxide.

16. The process as claimed in any one of claims 11 to 15, wherein the reaction mixture has a pH in the range of 8.0 to 12.5.

## Patentansprüche

1. Mundpflegezusammensetzung, die umfaßt:
a) von 10 bis 40 Gewichts-% Calciumsilikathydrat, umfassend von 20 bis 70% SiO₂, bezogen auf das Gewicht des Calciumsilikathydrats, und
b) physiologisch verträglichen Träger, umfassend Feuchthaltemittel, Tensid, Verdickungsmittel oder eine Mischung davon,
wobei das Calciumsilikathydrat in einer Menge von 5 bis 40% Hydratwasser, bezogen auf das Gewicht des Calciumsilikathydrats, umfasst.

2. Mundpflegezusammensetzung, wie im Anspruch 1 beansprucht, wobei das Calciumsilikathydrat teilchenförmig ist.

3. Mundpflegezusammensetzung, wie im Anspruch 1 oder Anspruch 2 beansprucht, wobei das Calciumsilikathydrat von 5 bis 40% CaO, bezogen auf das Gewicht des Calciumsilikathydrats, umfasst.

4. Mundpflegezusammensetzung, wie in irgendeinem der vorhergehenden Ansprüche beansprucht, wobei das Calciumsilikathydrat nicht mesoporös ist.

5. Mundpflegezusammensetzung, wie in irgendeinem der vorhergehenden Ansprüche beansprucht, wobei das Calciumsilikathydrat mindestens partiell kristallin ist.

6. Mundpflegezusammensetzung, wie in irgendeinem der vorhergehenden Ansprüche beansprucht, wobei die Zusammensetzung ein Zahnputzmittel, vorzugsweise eine Zahnpasta oder ein Mundwasser, ist.

7. Mundpflegezusammensetzung, wie in irgendeinem der vorhergehenden Ansprüche beansprucht, wobei das Calciumsilikathydrat Ca und Si in einem Atomverhältnis von 1:1,5 bis 1:3 umfasst.

8. Mundpflegezusammensetzung, wie in irgendeinem der vorhergehenden Ansprüche beansprucht, wobei die Mundpflegezusammensetzung eine Monophasen-Zusammensetzung darstellt und im Wesentlichen frei von Phosphat ist.

9. Mundpflegezusammensetzung nach irgendeinem der Ansprüche 1 bis 8 zur Verwendung in einem therapeutischen Verfahren zum Remineralisieren und/oder Aufhellen von Zähnen eines Individuums, umfassend den Schritt des Aufbringens der Mundpflegezusammensetzung auf einen oder mehrere Zähne des Individuums.

10. Mundpflegezusammensetzung zur Verwendung zum Remineralisieren und/oder Aufhellen von Zähnen eines Individuums nach Anspruch 9, wobei die Mundpflegezusammensetzung auf die Zähne ohne Auftragen von Phosphat auf die Zähne aufgetragen wird.

11. Verfahren zur Herstellung einer Mundpflegezusammensetzung, wie in irgendeinem der Ansprüche 1 bis 8 beansprucht, umfassend die Schritte:
i) Inkontaktbringen einer Quelle von Silikationen mit einer Quelle von Calciumionen in einem wässrigen Medium, um eine Reaktionsmischung zu bilden,
ii) Gewinnen von Calciumsilikathydrat aus der Reaktionsmischung,
iii) Kombinieren des Calciumsilikathydrats mit einem physiologisch verträglichen Träger.

12. Verfahren, wie im Anspruch 11 beansprucht, wobei die Reaktionsmischung mit einem Ca:Si-Atomverhältnis von 1:1,5 bis 1:3 gebildet wird.

13. Verfahren, wie im Anspruch 11 oder Anspruch 12 beansprucht, wobei die Quelle der Silikationen ein wasserlösliches Silikatsalz, vorzugsweise ein Alkalimetallsilikat, darstellt.

14. Verfahren, wie in irgendeinem der Ansprüche 11 bis 13 beansprucht, wobei die Quelle der Calciumionen aus unlöslichem Calciumsalz, schwer löslichem Calciumsalz oder einer Mischung davon ausgewählt wird.

15. Verfahren, wie im Anspruch 14 beansprucht, wobei die Quelle der Calciumionen Calciumhydroxid und/oder Calciumoxid darstellt.

16. Verfahren, wie in irgendeinem der Ansprüche 11 bis 15 beansprucht, wobei die Reaktionsmischung einen pH in dem Bereich von 8,0 bis 12,5 aufweist.

## Revendications

1. Composition pour le soin oral comprenant :
a) de 10 à 40 % en masse d'hydrate de silicate de calcium comprenant de 20 à 70 % de SiO₂ en masse de l'hydrate de silicate de calcium ; et
b) un support physiologiquement acceptable comprenant un humectant, un tensioactif, un épaississant ou un mélange de ceux-ci ;
dans laquelle l'hydrate de silicate de calcium comprend de l'eau d'hydratation dans une quantité de 5 à 40 % en masse de l'hydrate de silicate de calcium.

2. Composition pour le soin oral selon la revendication 1, dans laquelle l'hydrate de silicate de calcium est particulaire.

3. Composition pour le soin oral selon la revendication 1 ou la revendication 2, dans laquelle l'hydrate de silicate de calcium comprend de 5 à 40 % de CaO en masse de l'hydrate de silicate de calcium.

4. Composition pour le soin oral selon l'une quelconque des revendications précédentes, dans laquelle l'hydrate de silicate de calcium n'est pas mésoporeux.

5. Composition pour le soin oral selon l'une quelconque des revendications précédentes, dans laquelle l'hydrate de silicate de calcium est au moins partiellement cristallin.

6. Composition pour le soin oral selon l'une quelconque des revendications précédentes, dans laquelle la composition est un dentifrice, de préférence une pâte dentaire ou une eau de bouche.

7. Composition pour le soin oral selon l'une quelconque des revendications précédentes, dans laquelle l'hydrate de silicate de calcium comprend Ca et Si dans un rapport atomique de 1:1,5 à 1:3.

8. Composition pour le soin oral selon l'une quelconque des revendications précédentes, dans laquelle la composition pour le soin oral est une composition de monophase et est pratiquement exempte de phosphate.

9. Composition pour le soin oral selon l'une quelconque des revendications 1 à 8 pour une utilisation dans un procédé thérapeutique pour une reminéralisation et/ou un blanchiment des dents chez un individu comprenant l'étape d'application de la composition pour le soin oral à une ou plusieurs dents de l'individu.

10. Composition pour le soin oral pour une utilisation dans une reminéralisation et/ou un blanchiment des dents chez un individu, selon la revendication 9 dans laquelle la composition pour le soin oral est appliquée aux dents sans application de phosphate aux dents.

11. Procédé de fabrication d'une composition pour le soin oral selon l'une quelconque des revendications 1 à 8 comprenant les étapes de :
i) mise en contact d'une source d'ions silicate avec une source d'ions calcium dans un milieu aqueux pour former un mélange réactionnel ;
ii) récupération d'hydrate de silicate de calcium du mélange réactionnel ;
iii) combinaison de l'hydrate de silicate de calcium avec un support physiologiquement acceptable.

12. Procédé selon la revendication 11, dans lequel le mélange réactionnel est formé avec un rapport atomique Ca:Si de 1:1,5 à 1:3.

13. Procédé selon la revendication 11 ou la revendication 12, dans lequel la source d'ions silicate est un sel de silicate soluble dans l'eau, de préférence un silicate de métal alcalin.

14. Procédé selon l'une quelconque des revendications 11 à 13, dans lequel la source d'ions calcium est choisie parmi un sel de calcium insoluble, un sel de calcium peu soluble ou un mélange de ceux-ci.

15. Procédé selon la revendication 14, dans lequel la source d'ions calcium est l'hydroxyde de calcium et/ou l'oxyde de calcium.

16. Procédé selon l'une quelconque des revendications 11 à 15, dans lequel le mélange réactionnel présente un pH dans l'intervalle de 8,0 à 12,5.
